# FASCICULE DE BREVET EUROPEEN

(11) **EP 2 529 702 B1**
(45) Date de publication et mention de la délivrance du brevet: **08.07.2015**
(21) Numéro de dépôt: 12168336.1
(22) Date de dépôt: 16.05.2012
(51) Int. Cl.: A61F 2/28, A61F 2/30, A61L 27/00

(54) **Implant renforcé en céramique biocompatible et son procédé de fabrication**
Verstärktes Implantant aus biokompatibler Keramik und sein Herstellungsverfahren
Reinforced implant made of biocompatible ceramic and method for manufacturing same

(30) Priorité: 30.05.2011 FR 1154724
(43) Date de publication de la demande: 05.12.2012
(73) Titulaire: 3Dceram, 87000 Limoges (FR)
(72) Inventeur: Chaput, Christophe, 87410 Le Palais sur Vienne (FR); Gaignon, Richard, 91770 Saint-Vrain (FR); Brie, Joël, 87000 Limoges (FR)
(74) Mandataire: Chaillot, Geneviève

(56) Documents cités:
- WO-A1-00/71083
- WO-A1-95/07509
- WO-A2-2007/045000
- US-A1- 2006 224 242

## Description

La présente invention concerne le domaine des implants osseux, notamment des implants crâniens destinés à combler un défaut osseux dans le crâne d'un être humain ou d'un mammifère.

Le traitement des défauts osseux du crane est aujourd'hui un problème majeur en chirurgie maxillo-faciale et en neurochirurgie. Les défauts crâniens peuvent avoir diverses origines et surviennent notamment à la suite d'une anomalie développementale, d'une maladie osseuse ou de traumatismes ayant entrainé des fractures du crane. Ils peuvent également être la conséquence d'une opération de neurochirurgie, telle qu'une craniectomie décompressive pratiquée dans le cas d'oedèmes cérébraux post-traumatiques, ou encore une opération d'extraction d'une tumeur cérébrale.

Ces défauts osseux sont réparés au cours d'une opération de reconstruction du crane, appelée cranioplastie, lors de laquelle on insère et on fixe dans le crane un implant osseux destiné à combler le défaut osseux.

Le matériau idéal pour les implants de cranioplastie doit être résistant, biocompatible, pour éliminer le risque d'inflammation, de rejet et d'infection, et doit pouvoir s'intégrer à la structure osseuse vivante pour, au final, en faire partie. L'intégration de l'implant au sein du tissu natif se fait via un processus de migration de cellules osseuses, les ostéoblastes, dans l'implant, qui vont contribuer à la formation d'un nouveau tissu osseux.

A cet égard, plusieurs types de matériaux ont été utilisés pour fabriquer ces implants. On a ainsi couramment employé l'os lui-même en réalisant une autogreffe à partir d'autres structures osseuses du patient, telles que les côtes. Cette solution n'est pas sans inconvénient : elle présente des risques de morbidité au niveau du site donneur; le matériau n'est disponible qu'en quantité limitée et est soumis à des difficultés de conservation.

Les implants osseux artificiels constituent une alternative d'intérêt à l'utilisation de greffes osseuses. On a notamment utilisé des implants métalliques ou en matière plastique. Cependant, ces implants manquent souvent de biocompatibilité, et ont une faible ostéoconductivité, ce qui réduit leur capacité d'intégration au sein du tissu osseux existant.

Les céramiques sont également des matériaux avantageux pour la fabrication d'implants de cranioplastie. On connaît ainsi des implants en hydroxyapatite ou en phosphate de calcium, des matériaux choisis pour leur biocompatibilité et leur ostéoconductivité appropriées.

La demande de brevet WO2005/094730 A1, décrit un procédé de fabrication d'implant crânien en céramique poreuse. Après l'acquisition d'une image tridimensionnelle du crane du patient, on modélise le défaut osseux par conception assistée par ordinateur. On réalise alors, par prototypage rapide, un prototype d'implant, qui servira à fabriquer un moule en sulfate de calcium, en résine ou en caoutchouc de silicone. Enfin, on utilise le moule pour fabriquer l'implant en céramique poreuse.

Les céramiques telles que l'hydroxyapatite ou le triphosphate de calcium, ont des propriétés intéressantes pour la fabrication d'implants. Leur composition chimique, à base de phosphate de calcium, est proche de celle la matière osseuse, et leur confère une bonne biocompatibilité. De plus, les céramiques peuvent être fabriquées poreuses et donc avoir une bonne ostéoconductivité qui permet aux implants de bien s'intégrer au tissu osseux.

Un des inconvénients des implants en céramique est cependant leur fragilité, à la fois durant le processus de fabrication, et sur la pièce finie. Les implants crâniens sont des pièces qui ont fréquemment une forme de calotte avec une épaisseur relativement faible par rapport à leur surface. Ces implants sont donc soumis à des contraintes internes importantes qui s'exercent en particulier lors des traitements thermiques auxquels la pièce est soumise durant sa fabrication : le déliantage et le frittage. Elles conduisent alors à une déformation de l'implant, qui peuvent le rendre inutilisable, car inadapté à la forme du défaut osseux. Elles peuvent également provoquer l'apparition de zones de fragilité de l'implant susceptibles de favoriser sa rupture. Ces inconvénients rendent difficiles, voire impossible la fabrication d'implants en céramique de grande dimension, ce qui limite l'utilisation de ce matériau à la réparation de défauts osseux de faible dimension.

Aucun des documents de l'état antérieur de la technique ne divulgue de solution à ce problème de fragilité. Le document WO 00/71083 A1 divulgue un implant crânien étant fait de céramique biocompatible et comprenant un corps d'implant ayant une forme et des dimensions correspondant sensiblement à la forme et aux dimensions du défaut crânien à combler. Le document US2006/0224242 A1 décrit un implant chirurgical composite constitué d'un feuillet de résine thermoplastique et d'une combinaison d'un maillage métallique et de plaques métalliques. Le document WO 95/07509 A1 décrit un procédé de construction stéréolithographique de modèles incluant des prothèses. Le document WO 2007/045000 A2 divulgue des procédés, techniques, matériaux, dispositifs et utilisations pour la fabrication et l'ajustement d'implants biocompatibles.

La présente invention a donc pour objectif d'améliorer la résistance mécanique des implants crâniens en céramique, de manière à éviter la déformation et la fragilisation des implants au cours de leur fabrication. L'invention a également pour but d'améliorer la résistance d'implants crâniens en céramique et de permettre la fabrication d'implants crâniens en céramique de grande dimension.

A cet effet, la présente demande concerne un implant crânien destiné à combler un défaut crânien du crâne d'un mammifère ou d'un être humain, ledit implant crânien étant fait de céramique biocompatible et comprenant un corps d'implant ayant une forme et des dimensions correspondant sensiblement à la forme et aux dimensions du défaut crânien à combler, le corps d'implant comprenant une face externe tournée vers l'extérieur du crâne et une face interne tournée vers l'intérieur du crâne lorsque l'implant est en place sur le crâne, caractérisé par le fait que l'implant crânien comprend un ou plusieurs éléments de renfort faisant saillie sur la face interne du corps d'implant.

La présence du ou des éléments de renfort permet d'améliorer la résistance mécanique de l'implant à toutes les étapes de fabrication de la pièce. Ils permettent notamment d'éviter sa déformation lors du déliantage et du frittage ainsi que l'apparition de zones de fragilisation, telles que des microfissures dans l'implant.

Les éléments de renfort peuvent consister en toute structure en saillie apte à renforcer la résistance mécanique de l'implant. Ils peuvent notamment consister en des bandes de renfort, ou en tout autre élément géométrique.

La céramique est par exemple de l'alumine, une céramique à base de phosphate de calcium, telle que l'hydroxyapatite, le phosphate tricalcique, ou un mélange de celles-ci.

Le ou les éléments de renfort sont de préférence réalisés d'une seule pièce avec le corps de l'implant.

Cette conception est avantageuse par rapport à une conception consistant en un ou plusieurs éléments de renfort rapportés et fixés sur le corps de l'implant après la fabrication de celui-ci. Outre le gain de temps procuré par cette conception, on évite aussi le risque de mauvaise fixation et/ou de décollement du ou des éléments de renfort, tout en augmentant leur capacité de renfort.

Avantageusement, le ou les éléments de renfort font saillie sur une épaisseur inférieure à la distance séparant la face interne du corps d'implant de la face externe de la structure anatomique sous-jacente, telle que la dure-mère du mammifère ou de l'être humain recevant l'implant, lorsque l'implant est en place sur le crâne.

On évite ainsi de faire pression sur les structures anatomiques sous-jacentes, et notamment sur la face externe de la dure-mère du cerveau du mammifère ou de l'être humain destiné à recevoir l'implant.

Dans un mode de réalisation particulier, une pluralité d'ouvertures sont ménagées dans au moins un des éléments de renfort, les ouvertures étant ménagées sur la face supérieure du ou des éléments de renfort et/ou sur la ou les faces latérales du ou des éléments de renfort.

Ces ouvertures sont destinées à favoriser le déliantage de l'implant. Le déliantage consiste en un traitement thermique qui fait suite à la fabrication de l'implant à partir d'une composition contenant une résine polymérisable. Le déliantage sert à brûler la résine non polymérisée. Les ouvertures ménagées dans le ou les éléments de renfort ont pour fonction de faciliter la libération des vapeurs de résine non polymérisée, au cours du déliantage.

L'implant crânien comporte de préférence au moins deux régions dont les porosités sont différentes.

Par exemple, l'implant peut comporter au moins une région poreuse et au moins une région non poreuse.

La ou les régions poreuses sont destinées à être colonisées par les cellules osseuses telles que les ostéoblastes. Les pores sont ouvertes et de préférence interconnectées, et leurs dimensions sont choisies pour permettre une bonne pénétration des cellules osseuses dans celles-ci. Les régions non poreuses sont destinées à renforcer mécaniquement l'implant.

La conception d'un implant présentant des régions de porosité différente est innovante par rapport à l'état antérieur de la technique et nécessite une fabrication couche par couche, notamment par prototypage rapide. Cette conception n'est par exemple pas possible avec le procédé de moulage décrit dans la demande WO2005/094730 A1.

Le corps d'implant est de préférence poreux au voisinage d'une partie de la surface du corps d'implant et est non poreux au coeur du corps d'implant.

Il a en effet été constaté que, lors de l'intégration de l'implant au sein du tissu osseux, les cellules osseuses ne pénétraient pas dans l'épaisseur entière du corps d'implant. Il est donc avantageux de ne prévoir les régions poreuses qu'au voisinage de la surface du corps d'implant, et de prévoir une structure non poreuse au coeur du corps d'implant qui renforce la solidité de l'implant.

Le ou les éléments de renfort sont de préférence non poreux, ce qui augmente leur capacité à renforcer mécaniquement la pièce.

Plusieurs modes de fixation de l'implant au crâne peuvent être envisagés. Un mode de fixation possible consiste à fixer l'implant par des fils de suture.

A cet effet, le corps d'implant peut comprendre, au voisinage de sa périphérie, des ouvertures d'attache traversantes.

Ces ouvertures sont destinées à faire passer le fil de suture pour une fixation avec la région osseuse qui entoure le défaut crânien.

Les ouvertures d'attache sont de préférence entourées d'une région d'attache non poreuse, plus solide mécaniquement qu'une région poreuse.

Les éléments de renfort peuvent prendre différentes formes qui seront notamment choisies selon la forme de l'implant, selon la position du défaut crânien et selon les structures anatomiques sous-jacentes.

Ainsi, dans un mode de réalisation particulier l'implant crânien comprend plusieurs éléments de renfort dont au moins une partie consiste en des bandes de renfort et en un élément central, tel qu'un anneau ou un cercle, les bandes de renfort s'étendant en étoile vers la périphérie de l'implant à partir de l'élément central.

Dans un autre mode de réalisation, l'implant crânien comprend plusieurs éléments de renfort dont au moins une partie consiste en des bandes de renfort formant une grille.

L'invention concerne également un procédé de fabrication d'un implant crânien en céramique biocompatible caractérisé par le fait qu'il comprend les étapes suivantes:
- on fait l'acquisition d'une image tridimensionnelle du crâne d'un patient présentant un défaut crânien.
- on construit, par conception assistée par ordinateur, un modèle informatique de corps d'implant dont la forme correspond sensiblement au défaut crânien, et dont les dimensions sont légèrement plus grandes que le défaut crânien, de manière à prévoir un retrait de la céramique au cours de la fabrication de l'implant crânien;
- on ajoute à ce modèle informatique de corps d'implant, par conception assistée par ordinateur, des éléments de renfort, de façon à obtenir un modèle informatique d'implant crânien, les éléments de renfort faisant saillie sur la face interne du corps d'implant.
- on fabrique un implant crânien en céramique biocompatible par prototypage rapide à partir du modèle informatique d'implant crânien.

A titre d'exemple de technique de prototypage rapide, on peut citer la stéréolithographie.

Pour mieux illustrer l'objet de la présente invention, on va en décrire ci-après plusieurs modes de réalisation, avec référence aux dessins annexés.
La Figure 1 représente une vue en perspective obtenue par modélisation d'un crâne présentant un défaut crânien.
La Figure 2 représente une vue en perspective du modèle de crâne représenté en Figure 1 sur lequel est placé un implant crânien selon un premier mode de réalisation de l'invention.
La Figure 3 représente une vue en perspective obtenue par modélisation, d'un implant crânien selon un premier mode de réalisation de l'invention.
La Figure 4 représente une vue du dessus obtenue par modélisation de l'implant crânien de la Figure 3.
La Figure 5 représente une vue en perspective obtenue par modélisation d'un implant crânien selon un second mode de réalisation de l'invention.

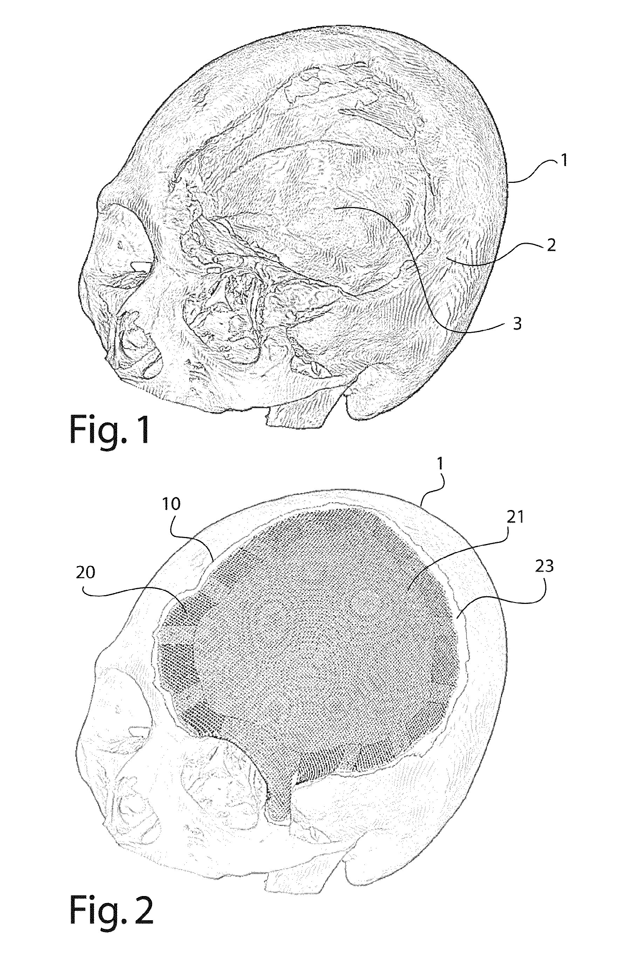

Si l'on se réfère à la Figure 1, on peut y voir représenté un crâne 1 selon une vue en perspective. Ce crâne présente un défaut crânien 2 très important. Le défaut crânien 2 prend la forme d'une calotte qui couvre une très large partie de l'os frontal et une partie de l'os pariétal du côté gauche et au centre du crâne. Le défaut s'étend frontalement jusqu'en bordure de l'orbite gauche du crâne et comprend une partie en forme de langue dépassant de la calotte et descendant sur le côté gauche de l'orbite gauche. Le défaut crânien laisse apparaître la dure-mère 3, membrane supérieure des méninges qui a notamment pour fonction de protéger le cerveau.

Sur la Figure 2 est représenté le crâne de la Figure 1 portant un implant crânien 10 selon un premier mode de réalisation de l'invention. Cet implant comprend un corps d'implant 20 qui comprend une face externe 21 tournée vers l'extérieur du crâne et une face interne, non visible sur la Figure 2 et tournée vers l'intérieur du crâne. La forme de l'implant reproduit sensiblement la forme du défaut crânien. L'implant a donc globalement la forme d'une calotte et comporte une partie en forme de langue qui vient épouser la partie correspondante du défaut crânien. Le corps d'implant 20 comprend, au niveau de sa face externe 21, une bordure d'appui 23 qui dépasse légèrement du défaut crânien. Cette bordure d'appui 23, plus fine que le reste du corps d'implant 20, s'étend sur la périphérie du corps de l'implant, dans le prolongement de sa face externe 21 et vient reposer sur les régions du crâne entourant le défaut crânien 2. Cette bordure d'appui 23 mise à part, les dimensions du corps d'implant 20 correspondent aux dimensions du défaut crânien 2, de telle sorte que l'implant 10 vienne exactement combler le défaut crânien.

Le corps d'implant 20 a une structure poreuse au voisinage de sa surface. Sur la Figure 2, cette structure poreuse est visible sur la face externe 21 du corps d'implant 20. Les pores sont destinés à favoriser l'infiltration et la colonisation de l'implant 10 par les cellules osseuses telles que les ostéoblastes. Leur dimension est donc prédéterminée et choisie pour laisser passer les cellules osseuses. A titre d'exemple, les pores ont une largeur de 500 microns. La structure poreuse prend ici la forme d'une grille avec des pores rectangulaires, mais l'homme du métier appréciera que la structure poreuse du corps d'implant peut avoir toute autre forme appropriée pour un passage des cellules. La bordure d'appui 23 est non poreuse, de façon à lui assurer une bonne résistance mécanique. L'implant 10 est fixé au crâne par des fils de sutures non représentés sur la Figure 2.

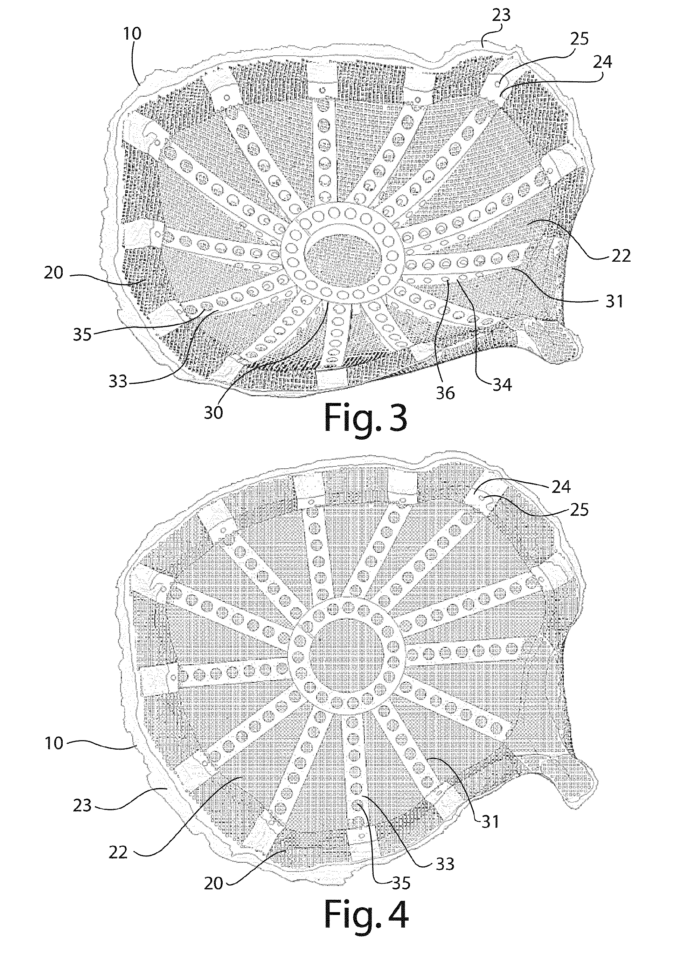

Si l'on se réfère aux Figures 3 et 4, on peut y voir représenté un implant crânien 10 selon un premier mode de réalisation de l'invention. Sur la Figure 3, l'implant 10 est représenté en perspective. L'implant comprend un corps d'implant 20 et des éléments de renfort 30, 31. Les éléments de renfort 30, 31 sont rapportés sur la face interne 22 du corps d'implant et fait saillie à partir de celle-ci. Les éléments de renfort 30, 31 prennent ici la forme d'un anneau circulaire 30 autour duquel des bandes de renfort 31 s'étendent en direction de la périphérie de l'implant. L'épaisseur des éléments de renfort 30, 31 est variable et est maximale au niveau de l'anneau 30, au voisinage du centre de l'implant 10 pour se réduire le long des bandes 31 en allant vers la périphérie de l'implant 10.

Des ouvertures borgnes 35 sont percées dans la face supérieure 33 des éléments de renfort 30 de façon à favoriser le déliantage de la pièce lors de sa fabrication. Elles mettent à jour la face interne 22 sous-jacente du corps d'implant 20. D'autres ouvertures 36, traversantes, sont percées dans les faces latérales 34 des bandes de renfort 31. Elles ont également la fonction de faciliter le déliantage. Chaque bande de renfort 31 s'étend en direction de la périphérie de l'implant jusqu'à une région d'attache 24 située au voisinage de cette périphérie. Chaque région d'attache 24 comprend une ouverture d'attache 25 traversante destinée à faire passer le fil de suture nécessaire à la fixation de l'implant 10 au crâne. De plus, une bordure d'appui 23 de fine épaisseur entoure l'implant. Comme indiqué précédemment, cette bordure dépasse légèrement du contour du défaut à combler et sert à faciliter la mise en place de l'implant 10 sur les régions du crâne entourant le défaut crânien.

Le corps d'implant 20 a une structure poreuse au voisinage de sa surface. Cette structure poreuse est visible sur la face interne 22 du corps d'implant 20. Comme indiqué précédemment pour la face externe, cette porosité a une architecture contrôlée et prend ici la forme d'une grille avec des pores rectangulaires. Bien que cela ne soit pas visible sur les Figures 3 et 4, le corps d'implant 20 n'est poreux qu'en surface et comprend un coeur à structure dense, c'est-à-dire non poreuse. De cette manière, les cellules osseuses peuvent infiltrer la surface du corps d'implant 20 de manière à favoriser l'intégration de l'implant à la structure osseuse. Par ailleurs, l'implant 10 reste solide grâce à son coeur non poreux. Bien qu'une majeure partie de la surface du corps d'implant 20 soit poreuse, les régions d'attache 24 sont conçues non poreuses de façon à ne pas être fragilisés. Les éléments de renforts 30, 31 et la bordure externe 23 sont également non poreux.

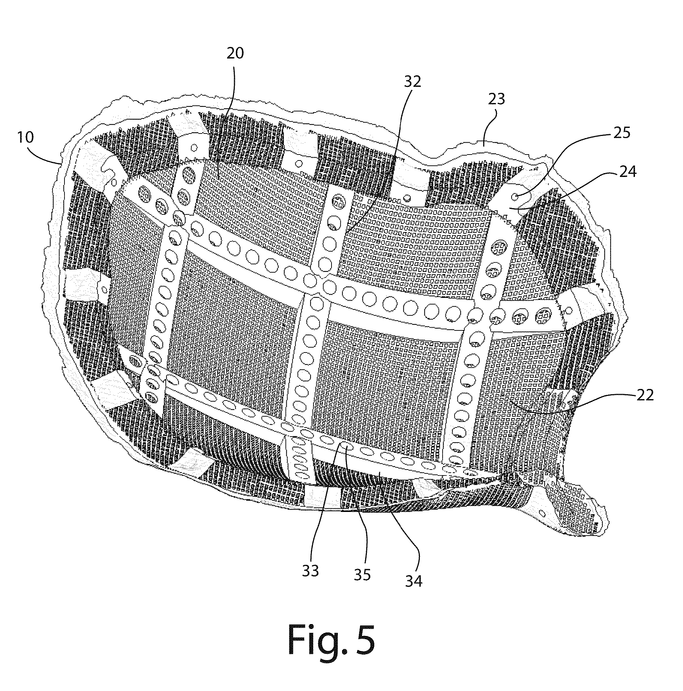

Si l'on se réfère à la Figure 5, on y voit représenté un implant crânien 10 selon un autre mode de réalisation de l'invention. Cet implant 10 diffère de l'implant représenté en Figure 3 et 4 par la structure des éléments de renfort 32. Sur la Figure 5, les éléments de renfort 32 prennent la forme d'une grille formée par des bandes de renfort 32 s'étendant d'une région voisine d'un bord de la face interne 22 du corps d'implant 20 à une région voisine d'un autre bord de la face interne 22 du corps d'implant 20.

On va maintenant décrire un exemple de procédé de fabrication d'un implant selon l'invention.

On commence par acquérir une image du crâne d'un patient par tomodensitométrie (« CT scan »). Cette image est alors traitée par conception assistée par ordinateur (CAO) de façon à construire un modèle informatique de corps d'implant. L'implant étant destiné à combler le défaut crânien, on donne au modèle de corps d'implant une forme qui correspond au morceau de crane manquant. Cependant, afin d'anticiper le retrait de la céramique, on augmente légèrement les dimensions du modèle de corps d'implant par rapport aux dimensions du défaut crânien. Le retrait de la céramique est un phénomène connu qui se produit lors du frittage des céramiques et aboutit à un rétrécissement de la pièce finie par rapport à la pièce crue. En prévoyant un modèle informatique et donc une pièce crue légèrement plus grande que le défaut à combler, on obtient une pièce finie bien ajustée au défaut.

Le modèle informatique de corps d'implant peut également intégrer d'autres caractéristiques telles que celles représentées présenté en Figures 3 et 4, ou en Figure 5. On peut notamment lui donner une architecture particulière de porosité, selon laquelle l'implant n'est poreux qu'au voisinage de sa surface et présente une structure dense dans sa partie centrale. La porosité peut avoir la forme présentée dans les Figure 3 à 5, à savoir un motif de grille avec des pores rectangulaires. Le modèle informatique de corps implant peut également inclure une bordure d'appui sur le contour périphérique du corps d'implant et destinée à reposer sur les régions du crâne entourant le défaut crânien.

On ajoute au modèle d'informatique de corps d'implant un ou plusieurs éléments de renfort pouvant par exemple prendre une forme d'étoile telle que représentée en Figures 3 et 4, ou une forme de grille telle que celle représentée en Figure 5. Ces éléments sont de préférence non poreux, et présentent des ouvertures transversales et des ouvertures latérales pour faciliter le déliantage.

D'une manière générale, l'épaisseur des éléments de renfort est choisie de façon à ce que les éléments de renfort ne fassent pas pression sur la dure-mère sous-jacente lorsque l'implant est en place sur le crâne. L'épaisseur maximale des éléments de renfort dépend donc de la position du défaut crânien à combler et pourra être déterminée selon le cas d'espèce par l'homme du métier.

On utilise le modèle informatique d'implant renforcé pour fabriquer un implant renforcé en céramique biocompatible par stéréolithographie en voie pâteuse, selon le procédé décrit dans la demande de brevet WO03/066326. Tout autre procédé de prototypage rapide est également adapté à cette fabrication.

On prépare pour cela une pâte ayant la composition suivante (en % de la masse totale)

| | |
|---|---|
| céramique | 80 |
| résine | 11,51 |
| photoinitiateur | 0, 09 |
| dispersant | 1,1 |
| plastifiant | 7,3 |

La céramique sera par exemple de l'hydroxyapatite. La résine pourra être une résine acrylate, telle que le diméthacrylate de bisphénol A di-éthoxylé ou le diacrylate de 1, 6 hexanediol. Le photoinitiateur sera choisi parmi les photoinitiateurs couramment utilisés dans la polymérisation des acrylates. On citera notamment le 2,2'-diméthoxy-2-phénylacétophénone et le 2-hydroxy-2-méthyl-1-phényl-propane-1-one. Le dispersant est avantageusement un ester phosphorique. Comme plastifiant on peut choisir un ou plusieurs agents du groupe constitué par la famille des glycols (ex : le polyéthylène glycol), la famille des phtalates (ex : le dibutylphtalate), le glycérol.

On utilise cette pâte pour fabriquer, par stéréolithographie, un implant crânien cru tel que celui représenté aux Figure 3 et 4. La fabrication est faite en 500 couches de 100 microns.

On soumet l'implant crânien cru à un traitement thermique (déliantage) jusqu'à 600°C avec un palier de 2h à 600°C puis à un frittage jusqu'à 1250°C avec un palier de 1h30 à 1250°C. La pièce finie présente une dimension de 130mm*110mm*40mm. L'épaisseur maximale des éléments de renfort 30, 31 est de 5 mm, atteinte au niveau de l'anneau central 30. Les bandes de renfort 31 ont une largeur de 6 mm, les ouvertures 35 dans la face supérieure 33 des éléments de renfort 30, 31 ont un diamètre de 3mm, et les ouvertures 36 dans les faces latérales 34 des éléments de renfort ont un diamètre de 2 mm.

On constate que l'implant renforcé fini n'est pas déformé par rapport au modèle informatique d'implant. Sa forme et ses dimensions correspondent donc à celles du défaut crânien que l'on veut combler. Il ne présente pas non plus de défauts mécaniques tels que des fissurations susceptibles de fragiliser l'implant, et présente une meilleure résistance qu'un implant non renforcé.

## Revendications

1. Implant crânien (10) destiné à combler un défaut crânien (2) du crâne (1) d'un mammifère ou d'un être humain, ledit implant crânien (10) étant fait de céramique biocompatible et comprenant un corps d'implant (20) ayant une forme et des dimensions correspondant sensiblement à la forme et aux dimensions du défaut crânien (2) à combler, le corps d'implant comprenant une face externe (21) tournée vers l'extérieur du crâne et une face interne (22) tournée vers l'intérieur du crâne (1) lorsque l'implant est en place sur le crâne (1), **caractérisé par le fait que** l'implant crânien (10) comprend un ou plusieurs éléments de renfort (30, 31, 32) faisant saillie sur la face interne (22) du corps d'implant (20).

2. Implant crânien (10) selon la revendication 1, **caractérisé par le fait que** le ou les éléments de renfort (30, 31, 32) sont réalisés d'une seule pièce avec le corps de l'implant (20).

3. Implant crânien (10) selon l'une des revendications 1 et 2, **caractérisé par le fait que** le ou les éléments de renfort (30, 31, 32) font saillie sur une épaisseur inférieure à la distance séparant la face interne (22) du corps d'implant (20) de la face externe de la structure anatomique sous-jacente, telle que la dure-mère (3) du mammifère ou de l'être humain recevant l'implant (10), lorsque l'implant est en place sur le crâne (1).

4. Implant crânien (10) selon l'une des revendications 1 à 3, **caractérisé par le fait qu'**une pluralité d'ouvertures (35, 36) sont ménagées dans au moins un des éléments de renfort (30, 31, 32), les ouvertures (35, 36) étant ménagées sur la face supérieure (33) du ou des éléments de renfort (30, 31, 32) et/ou sur la ou les faces latérales (34) du ou des éléments de renfort (30, 31, 32).

5. Implant crânien (10) selon l'une des revendications 1 à 4, **caractérisé par le fait que** l'implant crânien (10) comporte au moins deux régions dont les porosités sont différentes.

6. Implant crânien selon la revendication 5, **caractérisé par le fait que** le corps d'implant (20) est poreux au voisinage d'une partie de la surface du corps d'implant (20) et est non poreux au coeur du corps d'implant (20).

7. Implant crânien selon la revendication 5, **caractérisé par le fait que** le ou les éléments de renfort (30, 31, 32) sont non poreux.

8. Implant crânien (10) selon l'une des revendications 1 à 7, **caractérisé par le fait que** le corps d'implant (20) comprend, au voisinage de sa périphérie, des ouvertures d'attache (25) traversantes, de préférence entourées d'une région d'attache (24) non poreuse.

9. Implant crânien (10) selon l'une des revendications 1 à 8, **caractérisé par le fait que** l'implant crânien (10) comprend plusieurs éléments de renfort (30, 31) dont au moins une partie consiste en des bandes de renfort (31) et en un élément central (30), tel qu'un anneau ou un cercle, les bandes de renfort (31) s'étendant en étoile vers la périphérie de l'implant à partir de l'élément central (30).

10. Implant crânien (10) selon l'une des revendications 1 à 8, **caractérisé par le fait que** l'implant crânien (10) comprend plusieurs éléments de renfort (32) dont au moins une partie consiste en des bandes de renfort (32) formant une grille.

11. Procédé de fabrication d'un implant crânien (10) en céramique biocompatible, **caractérisé par le fait qu'**il comprend les étapes suivantes:
- on fait l'acquisition d'une image tridimensionnelle du crâne d'un patient présentant un défaut crânien ;
- on construit, par conception assistée par ordinateur, un modèle informatique de corps d'implant (20) dont la forme correspond sensiblement au défaut crânien (2), et dont les dimensions sont légèrement plus grandes que le défaut crânien (2), de manière à prévoir un retrait de la céramique au cours de la fabrication de l'implant crânien (10);
- on ajoute à ce modèle informatique de corps d'implant (20), par conception assistée par ordinateur, des éléments de renfort (30, 31, 32), de façon à obtenir un modèle informatique d'implant crânien (10), les éléments de renfort (30, 31, 32) faisant saillie sur la face interne (22) du corps d'implant (20) ;
- on fabrique un implant crânien (10) en céramique biocompatible par prototypage rapide à partir du modèle informatique d'implant crânien.

## Patentansprüche

1. Schädelimplantat (10) zum Füllen eines Schädeldefekts (2) des Schädels (1) eines Säugetiers oder eines Menschen, wobei das Schädelimplantat (10) aus einer biokompatiblen Keramik gefertigt ist und einen Implantatkörper (20) umfasst, der eine Form und Abmessungen aufweist, die im Wesentlichen der Form und den Abmessungen des zu füllenden Schädeldefekts (2) entsprechen, wobei der Implantatkörper eine Außenfläche (21), die vom Schädel aus nach außen gerichtet ist, und eine Innenfläche (22) umfasst, die ins Innere des Schädels (1) gerichtet ist, wenn sich das Implantat an der richtigen Stelle am Schädel (1) befindet, **dadurch gekennzeichnet, dass** das Schädelimplantat (10) ein oder mehrere Verstärkungselemente (30, 31, 32) umfasst, die an der Innenfläche (22) des Implantatkörpers (20) vorstehen.

2. Schädelimplantat (10) nach Anspruch 1, **dadurch gekennzeichnet, dass** das oder die Verstärkungselemente (30, 31, 32) einstückig mit dem Implantatkörper (20) ausgeführt ist bzw. sind.

3. Schädelimplantat (10) nach Anspruch 1 oder 2, **dadurch gekennzeichnet, dass** das oder die Verstärkungselemente (30, 31, 32) über eine Höhe vorsteht bzw. vorstehen, die kürzer ist als der Abstand zwischen der Innenfläche (22) des Implantatkörpers (20) und der Außenfläche der darunter befindlichen anatomischen Struktur, beispielsweise der Dura mater (3) des Säugetiers oder des Menschen, das bzw. der das Implantat (10) erhält, wenn sich das Implantat an der richtigen Stelle am Schädel (1) befindet.

4. Schädelimplantat (10) nach einem der Ansprüche 1 bis 3, **dadurch gekennzeichnet, dass** eine Mehrzahl von Öffnungen (35, 36) in mindestens einem der Verstärkungselemente (30, 31, 32) vorgesehen ist, wobei die Öffnungen (35, 36) an der Oberseite (33) des oder der Verstärkungselemente (30, 31, 32) und/oder an der oder den Seitenflächen (34) des oder der Verstärkungselemente (30, 31, 32) vorgesehen sind.

5. Schädelimplantat (10) nach einem der Ansprüche 1 bis 4, **dadurch gekennzeichnet, dass** das Schädelimplantat (10) mindestens zwei Bereiche aufweist, deren Porosität sich unterscheidet.

6. Schädelimplantat nach Anspruch 5, **dadurch gekennzeichnet, dass** der Implantatkörper (20) in der Nähe eines Abschnitts der Oberfläche des Implantatkörpers (20) porös ist und im Inneren des Implantatkörpers (20) nicht porös ist.

7. Schädelimplantat nach Anspruch 5, **dadurch gekennzeichnet, dass** das oder die Verstärkungselemente (30, 31, 32) nicht porös ist bzw. sind.

8. Schädelimplantat (10) nach einem der Ansprüche 1 bis 7, **dadurch gekennzeichnet, dass** der Implantatkörper (20) in der Nähe seines Rands durchgehende Befestigungsöffnungen (25) umfasst, die vorzugsweise von einem nicht porösen Befestigungsbereich (24) umgeben sind.

9. Schädelimplantat (10) nach einem der Ansprüche 1 bis 8, **dadurch gekennzeichnet, dass** das Schädelimplantat (10) mehrere Verstärkungselemente (30, 31) umfasst, von denen mindestens ein Teil aus Verstärkungsstreifen (31) und aus einem mittigen Element (30) wie einem Ring oder Kreis besteht, wobei die Verstärkungsstreifen (31) von dem mittigen Element (30) aus sternförmig in Richtung des Rands des Implantats verlaufen.

10. Schädelimplantat (10) nach einem der Ansprüche 1 bis 8, **dadurch gekennzeichnet, dass** das Schädelimplantat (10) mehrere Verstärkungselemente (32) umfasst, von denen mindestens ein Teil aus Verstärkungsstreifen (32) besteht, die ein Gitter bilden.

11. Verfahren zur Herstellung eines Schädelimplantats (10) aus einer biokompatiblen Keramik, **dadurch gekennzeichnet, dass** es folgende Schritte umfasst:
- Aufnehmen eines dreidimensionalen Bilds des Schädels eines Patienten mit einem Schädeldefekt;
- rechnergestütztes Konstruieren eines Computermodells des Implantatkörpers (20), dessen Form im Wesentlichen dem Schädeldefekt (2) entspricht und dessen Abmessungen etwas größer als der Schädeldefekt (2) sind, damit eine Schrumpfung der Keramik während der Herstellung des Schädelimplantats (10) einkalkuliert wird;
- mittels computergestütztem Konstruieren Hinzufügen von Verstärkungselementen (30, 31, 32) zu diesem Computermodell des Implantatkörpers (20), damit ein Computermodell des Schädelimplantats (10) erhalten wird, wobei die Verstärkungselemente (30, 31, 32) an der Innenfläche (22) des Implantatkörpers (20) vorstehen;
- Fertigen eines Schädelimplantats (10) aus biokompatibler Keramik mittels Rapid Prototyping auf der Basis des Computermodells des Schädelimplantats.

## Claims

1. A cranial implant (10) designed to fill a cranial defect (2) of the skull (1) of a mammal or a human, wherein said cranial implant (10) is made of biocompatible ceramic and comprises an implant body (20) having a shape and size substantially matching the shape and size of the cranial defect (2) to be filled, wherein the implant body comprises an outer face (21) facing outside the skull and an inner face (22) facing inside the skull (1) when the implant is disposed on the skull (1), **characterized in that** the cranial implant (10) comprises one or more reinforcing members (30, 31, 32) protruding from the inner face (22) of the implant body (20).

2. A cranial implant (10) according to claim 1, **characterized in that** the one or more reinforcing members (30, 31, 32) are integrally made with the implant body (20).

3. A cranial implant (10) according to claim 1 or 2, **characterized in that** the one or more reinforcing members (30, 31, 32) protrude over a thickness smaller than the distance separating the inner face (22) of the implant body (20) from the outer face of the underlying anatomic structure, such as the dura mater of the mammal or the human the receiving the implant (10), when the implant is disposed on the skull (1).

4. A cranial implant (10) according to anyone of claims 1 to 3, **characterized in that** a plurality of openings (35, 36) are provided in at least one of the reinforcing members (30, 31, 32), wherein the openings (35, 36) are provided on the upper face (33) of the one or more reinforcing members (30, 31, 32) and/or on at least one side face (34) of the one or more reinforcing members (30, 31, 32).

5. A cranial implant (10) according to anyone of claims 1 to 4, **characterized in that** the cranial implant (10) comprises at least two areas whose porosities are different.

6. A cranial implant according to claim 5, **characterized in that** the implant body (20) is porous in the vicinity of part of the surface of the implant body (20) and is non-porous at the core of the implant body (20).

7. A cranial implant according to claim 5, **characterized in that** the one or more reinforcing members (30, 31, 32) are non-porous.

8. A cranial implant (10) according to anyone of claims 1 to 7, **characterized in that** the implant body (20) comprises, in the vicinity of the periphery thereof, attachment through openings (25), preferably surrounded by a non-porous attachment area (24).

9. A cranial implant (10) according to anyone of claims 1 to 8, **characterized in that** the cranial implant (10) comprises several reinforcing members (30, 31) of which at least a part consists of reinforcing strips (31) and a central member (30), such as a ring or a circle, wherein the reinforcing strips (31) extend star-like to the periphery of the implant from the central member (30).

10. A cranial implant (10) according to anyone of claims 1 to 8, **characterized in that** the cranial implant (10) comprises several reinforcing members (32) of which at least a part consists of reinforcing strips (32) forming a grid.

11. A method for manufacturing a biocompatible ceramic cranial implant (10), **characterized in that** it comprises the following steps:
- acquiring a three dimensional image of the skull of a patient having a cranial defect;
- building, by computer-aided design, an implant body (20) computer model whose shape substantially matches the cranial defect (2) and whose size is slightly larger than the cranial defect (2), so as to anticpiate shrinking of the ceramic during manufacturing of the cranial implant (10);
- adding reinforcing members (30, 31, 32), by computer-aided design, to this implant body (20) ocmputer model, so as to obtain a cranial implant (10) computer model, wherein the reinforcing members (30, 31, 32) protrude from the inner face (22) of the implant body (20);
- manufacturing a biocompatible ceramic cranial implant (10) by rapid prototyping using the cranial implant computer model.
